(19) Europäisches Patentamt
European Patent Office
Office européen des brevets

(11) EP 4 806 415 A1

(12) # EUROPEAN PATENT APPLICATION
published in accordance with Art. 153(4) EPC

(43) Date of publication:
**16.09.2026 Bulletin 2026/38**

(21) Application number: **24888735.8**

(22) Date of filing: **06.11.2024**

(51) International Patent Classification (IPC):
***A61K 6/62*** *(2020.01)* ***A61C 13/15*** *(2006.01)*
***A61C 13/087*** *(2006.01)* ***A61K 6/60*** *(2020.01)*
***B29C 64/129*** *(2017.01)* ***B29C 64/314*** *(2017.01)*
***B33Y 70/00*** *(2020.01)* ***C08F 290/06*** *(2006.01)*
***C08F 299/06*** *(2006.01)*

(52) Cooperative Patent Classification (CPC):
**A61C 13/087; A61C 19/003; A61K 6/60; A61K 6/62; B29C 64/129; B29C 64/314; B33Y 70/00; C08F 290/06; C08F 299/06**

(86) International application number:
**PCT/JP2024/039441**

(87) International publication number:
**WO 2025/100441 (15.05.2025 Gazette 2025/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.11.2023 JP 2023190437**

(71) Applicant: **Kuraray Noritake Dental Inc.**
**Kurashiki-shi, Okayama 710-0801 (JP)**

(72) Inventor: **SUZUKI Kenji**
**Tainai-shi, Niigata 959-2653 (JP)**

(74) Representative: **D Young & Co LLP**
**3 Noble Street**
**London EC2V 7BQ (GB)**

(54) **STEREOLITHOGRAPHIC RESIN COMPOSITION**

(57) The present invention provides a stereolithographic resin composition that possesses excellent shape accuracy and exhibits superior strength, toughness, and water resistance in the shaped article while reducing leachables from the shaped article. The present invention relates to a stereolithographic resin composition comprising a polyfunctional (meth)acrylic polymerizable compound (A), and a photopolymerization initiator containing four or more heteroatoms within a single molecule (B). Preferably, the stereolithographic resin composition further comprises a monofunctional (meth)acrylic polymerizable compound (C). Preferably, the polyfunctional (meth)acrylic polymerizable compound (A) comprises a polyfunctional (meth)acrylic polymerizable compound (A)-I having a molecular weight of less than 500.

EP 4 806 415 A1

## Description

### TECHNICAL FIELD

The present invention relates to a stereolithographic resin composition. More specifically, the invention relates to a stereolithographic material for dental use that possesses excellent shape accuracy and exhibits superior strength, toughness, and water resistance in the shaped article while achieving excellent biological safety with reduced amounts of leachables from the shaped article. The material is especially suited for applications such as dental occlusal splints, denture base materials, and appliances for the treatment of sleep apnea.

### BACKGROUND ART

Various proposals have been made concerning optical 3D fabrication, a method that produces three-dimensional shaped articles through a repeated procedure whereby a liquid photocurable resin is cured into a thin layer under controlled application of necessary amounts of light energy, and another thin layer is cured on the cured layer under controlled application of light after supplying another portion of the liquid photocurable resin onto the previously formed cured layer.

Vat stereolithography is a technique typically used for optical fabrication of three-dimensional shaped articles. In this technique, a liquid photocurable resin composition is placed in a vat, and a computer-controlled ultraviolet laser is selectively applied to the surface of the liquid photocurable resin composition to cure it to a predetermined thickness, forming a cured layer with the desired pattern. Continuously, another cured layer is formed on the cured layer by applying an ultraviolet laser in the same manner to the liquid photocurable resin composition supplied onto the previously cured layer in an amount necessary to form a single layer. This layering process is repeated until it produces the final three-dimensional shaped article. In recent years, this technique has attracted great interest because it enables easy and precision production of the desired three-dimensional shaped article in a relatively short time period, even when the article has a very complex shape.

Three-dimensional shaped articles created through stereolithography are expanding their applications from mere concept models to test models, prototypes, and final products. The field of dental materials is thought to greatly benefit from stereolithography because dental occlusal splints, denture base materials, and mouthpiece-like materials used for treating sleep disorders (for example, appliances for preventing bruxism or treating sleep apnea) require shapes that vary from patient to patient, aside from being complex in shape.

Some dental occlusal splints are fitted to adjust tooth alignment or jaw position as in so-called orthodontic mouthpieces or aligners while others are attached to teeth to reduce tooth wear due to clenching. Mouthguards are another type of splints that are worn in the mouth to protect the stomatognathic system and the brain by reducing injuries caused when large external forces are applied to teeth and jawbones during sports activities in contact sports. In orthodontics, the use of these devices has gained wide popularity over the last years due to their favorable aesthetics and convenient removability.

Denture base materials are materials used for the gum as a part of a denture attached to replace missing teeth. The demand for dentures has rapidly increased in recent years because of increasing ageing populations.

Appliances for the treatment of sleep apnea include appliances (oral appliances, or OA) attached to teeth during sleep for the treatment of obstructive sleep apnea syndrome (OSAS), and its use has been rapidly increasing.

Common requirements for dental occlusal splints, denture base materials, and OA include shape accuracy, strength, toughness, and water resistance. Low shape accuracy results in poor fit, leading to not only reduced comfort but also failure to provide the intended functional performance. Low strength creates discomfort by increasing flexure or deformation during wear, whereas a loss of toughness necessitates frequent replacement as it increases susceptibility to breakage from biting forces or deformation during wear. A loss of water resistance causes a reduction of mechanical properties during use, making the appliance practically useless by making the appliance easily deformable or breakable while being worn.

Another consideration is that fabrication of dental occlusal splints, denture base materials, and appliances for the treatment of sleep apnea typically requires taking an impression of the oral cavity. This is seen as a problem because the procedure involves discomfort, and places a burden on patients, in addition to requiring high technical skills.

Recent advances in digital technology have led to approaches that make use of an intraoral optical scan for taking an oral impression, and there have been attempts to apply stereolithography techniques for shaping. For fabrication, a stereolithographic resin composition is used. As a rule, resin compositions that exhibit strength tend to be more brittle, which makes it difficult to provide toughness, namely flexibility.

The difficulty in achieving both strength and toughness, combined with the need for shapeability and water resistance, makes it highly challenging to develop products that satisfy all of shape accuracy, strength, toughness, and water resistance.

Against this backdrop, techniques are available that can yield cured products having superior strength, toughness, and water resistance, as described in, for example, Patent Literatures 1 and 2. Patent Literatures 1 and 2 disclose examples in which a specific polyfunctional methacrylate and a monofunctional acrylate having multiple aromatic rings, representing polymerizable compounds contained as essential components, are combined with another essential component 2,4,6-trimethylbenzoyldiphenylphosphine oxide contained as a photopolymerization initiator.

CITATION LIST

Patent Literature

Patent Literature 1: JP2020-158417 A
Patent Literature 2: WO2021/162007

SUMMARY OF INVENTION

Technical Problem

However, examination by the present inventor revealed that Patent Literatures 1 and 2 do not describe anything about leachability concerning a tendency of degradation products of the photopolymerization initiator or unpolymerized components to leach, though the stereolithographic resin compositions are described as having a certain level of shape accuracy and exhibiting some degree of strength, toughness, and water resistance in the cured product. Through further investigation, the present inventor has found that these inventions have areas for improvement with respect to leachability.

The present inventor has also found that providing toughness, or flexibility, to the cured product of the stereolithographic resin composition alongside strength necessitates changes to the monomer, and, because monomers used to impart flexibility possess more flexible molecule structures that permit easier moisture penetration, it leads to a major drawback involving not only a reduction in strength but also increased leachability causing more release of degradation products of the photopolymerization initiator or unpolymerized components.

Stereolithographic materials, in particular, involve an extremely short irradiation time compared to non-stereolithographic dental materials, necessitating the incorporation of a large amount of photopolymerization initiator.

For example, dental filling materials and dental resin cements, which are widely available and commonly used as photocurable dental materials with the direct method involving curing within the oral cavity, are typically irradiated with light for about 10 to 30 seconds using a dental photoirradiator. When used with the indirect method, which cures the material extraorally before it is used in the oral cavity, the material is typically irradiated for 60 seconds to several minutes using an irradiator intended for dental laboratory use.

In contrast, in stereolithography using, for example, a vat stereolithography apparatus, the duration of photoirradiation is generally from one-tenth of these numbers to several seconds. Correspondingly, stereolithographic materials contain a total of 1 mass% to 5 mass% of photopolymerization initiator, as opposed to about 0.1 mass% to 0.5 mass% in dental filling materials or dental resin cements.

As described above, the duration of irradiation in stereolithographic materials is about one-tenth of that employed in traditional dental materials, increasing the content of photopolymerization initiator by a factor of approximately 10. This makes the issue of leaching from the cured product even more pronounced compared with traditional dental materials, making it particularly difficult to reduce leaching from the shaped article while satisfying shape accuracy, strength, toughness, and water resistance.

As described above, stereolithographic materials involve a particularly short irradiation time compared to dental materials not intended for stereolithography, necessitating the incorporation of a large amount of photopolymerization initiator. Solving the problem of leaching from the cured product has therefore been identified as a particularly important issue in stereolithographic materials.

However, reducing the content of photopolymerization initiator to reduce degradation products from the photopolymerization initiator results in deterioration of performance such as strength. This is incompatible with the inherent requirement of stereolithographic materials to incorporate a large amount of photopolymerization initiator due to the extremely short irradiation time. Consequently, it has been extremely difficult to satisfy all of shape accuracy, strength, toughness, and water resistance while reducing leaching from the shaped article.

It is accordingly an object of the present invention to provide a stereolithographic resin composition that possesses excellent shape accuracy and exhibits superior strength, toughness, and water resistance in the shaped article while reducing leachables from the shaped article.

Solution to Problem

Specifically, the present invention includes the following.

[1] A stereolithographic resin composition comprising a polyfunctional (meth)acrylic polymerizable compound (A), and a photopolymerization initiator containing four or more heteroatoms within a single molecule (B),
wherein the content of the photopolymerization initiator containing four or more heteroatoms within a single molecule (B) is 0.75 to 3.0 parts by mass relative to total 100 parts by mass of polymerizable compounds.
[2] The stereolithographic resin composition according to [1], which further comprises a monofunctional (meth)acrylic polymerizable compound (C).
[3] The stereolithographic resin composition according to [1] or [2], wherein the polyfunctional (meth)acrylic polymerizable compound (A) comprises a polyfunctional (meth)acrylic polymerizable compound (A)-I having a molecular weight of less than 500.
[4] The stereolithographic resin composition according to any one of [1] to [3], wherein the polyfunctional (meth)acrylic polymerizable compound (A) comprises a polyfunctional (meth)acrylic polymerizable compound (A)-II having a molecular weight of 500 or more.
[5] The stereolithographic resin composition according to any one of [1] to [4], wherein the polyfunctional (meth)acrylic polymerizable compound (A) comprises a polyfunctional (meth)acrylic polymerizable compound (A)-I having a molecular weight of less than 500, and a polyfunctional (meth)acrylic polymerizable compound (A)-II having a molecular weight of 500 or more.
[6] The stereolithographic resin composition according to any one of [1] to [5], wherein the polyfunctional (meth)acrylic polymerizable compound (A) comprises a polyfunctional (meth)acrylic polymerizable compound (A)-I-1 having a molecular weight of less than 500 and containing a urethane bond, and/or a (meth)acrylic polymerizable compound (A)-II-1 having a molecular weight of 500 or more and containing a urethane bond.
[7] The stereolithographic resin composition according to any one of [1] to [6], wherein the photopolymerization initiator (B) has a molecular weight of 400 or more.
[8] The stereolithographic resin composition according to any one of [1] to [7], wherein the photopolymerization initiator (B) comprises two or more carbonyl groups within a single molecule.
[9] The stereolithographic resin composition according to any one of [1] to [8], wherein the photopolymerization initiator (B) comprises bis(2,4,6-trimethylbenzoyl)phenylphosphine oxide.
[10] A dental material comprising a shaped article of the stereolithographic resin composition of any one of [1] to [9].
[11] A denture base material comprising a shaped article of the stereolithographic resin composition of any one of [1] to [9].
[12] A dental occlusal splint comprising a shaped article of the stereolithographic resin composition of any one of [1] to [9].
[13] A material for treating sleep disorder comprising a shaped article of the stereolithographic resin composition of any one of [1] to [9].
[14] A method for stereolithographically producing a three-dimensional shaped article using the stereolithographic resin composition of any one of [1] to [9].

Advantageous Effects of Invention

According to the present invention, a stereolithographic resin composition can be provided that possesses excellent shape accuracy and exhibits superior strength, toughness, and water resistance in the shaped article while reducing leachables from the shaped article. This makes a stereolithographic resin composition of the present invention suited for dental materials such as denture base materials and dental occlusal splints, as well as materials used for appliances for the treatment of sleep apnea.

DESCRIPTION OF EMBODIMENTS

The following describes the present invention in detail through embodiments.
In this specification, the upper limits and lower limits of numeric ranges (for example, ranges of contents of components, ranges of values calculated from components, and ranges of physical properties) can be appropriately combined. For example, in this specification, the lower and upper limits of numeric ranges presented in a stepwise fashion may be combined independently. As an example, in a statement such as "preferably 0.2 to 8.0 mass%, more preferably 0.5 to 5.0 mass%" for the same parameter, it is possible to combine the preferred lower limit (0.2 mass%) with the more preferred upper limit (5.0 mass%) to form a range of "0.2 to 5.0 mass%", or, alternatively, "0.5 to 8.0 mass%."
Concerning numeric ranges, it is also possible to define only the lower limit without specifying the upper limit, for

example, such as "12 mass% or more" or "12.5 mass% or more", based on a statement "more preferably 12 to 28 mass%, even more preferably 12.5 to 25 mass%". Similarly, it is possible to define only the upper limit without specifying the lower limit, such as "28 mass% or less" or "25 mass% or less."

Unless specifically indicated otherwise, when the expression of a numeric range is simply denoted as "30 to 70", it indicates 30 or more and 70 or less. Furthermore, for example, when a numeric range is "25 mass% or more and 75 mass% or less", the boundary values of the numeric range may be chosen from any of "more than 25 mass%", "25 mass% or more", "75 mass% or less", and "less than 75 mass%."

Similarly, for example, from statements indicated for the same parameter such as "more preferably 0.05 parts or more by mass, even more preferably 0.1 parts or more by mass" and "more preferably 15 parts or less by mass, even more preferably 10 parts or less by mass", it is possible to combine the more preferred lower limit (0.05 parts or more by mass) with the even more preferred upper limit (10 parts or less by mass) to form the range of "0.05 parts or more by mass and 10 parts or less by mass." Furthermore, as with the foregoing, it is also possible to specify only the lower limit as "0.05 parts or more by mass" or "0.1 parts or more by mass", and, similarly, only the upper limit as "15 parts or less by mass" or "10 parts or less by mass."

The numeric ranges described above are merely examples using mass percentages and parts by mass, and the same applies to various parameters, including molecular weight, flexural strength, and flexural modulus.

The present invention encompasses embodiments combining all or part of the embodiments described in this specification, provided that the present invention can exhibit its effects with such combinations made in various forms within the technical idea of the present invention.

As used herein, the term "polymerizable compound" is used to refer to a polymerizable compound polymerized by the photopolymerization initiator (B) described below.

As used herein, the term "(meth)acryl" is intended to be inclusive of both methacryl and acryl, and the same applies to similar expressions such as "(meth)acrylate", "(meth)acrylic acid ester", "(meth)acrylamide", and "(meth) acryloyloxy."

As used herein, the term "(meth)acrylic polymerizable compound" is intended to be inclusive of both polymerizable compounds having a (meth)acryloxy group as a polymerizable group, and polymerizable compounds having a (meth) acrylamide group as a polymerizable group.

As used herein, "polyfunctional" means that the number of polymerizable groups (for example, (meth)acryl groups) is more than one.

As used herein, "monofunctional" means having one polymerizable group (for example, a (meth)acryl group)

As used herein, "molecular weight" refers to a single value calculated from atomic weights when an oligomer or polymer structure is absent. When an oligomer or polymer structure is present, the term "molecular weight" refers to, unless otherwise specified, the weight-average molecular weight determined by gel permeation chromatography (GPC), expressed in terms of polystyrene equivalents.

As used herein, the term "heteroatom" as contained in photopolymerization initiator (B) refers to any atom other than carbon and hydrogen.

A stereolithographic resin composition of the present invention comprises a polyfunctional (meth)acrylic polymerizable compound (A), and a photopolymerization initiator containing four or more heteroatoms within a single molecule (B).

[Polyfunctional (Meth)Acrylic Polymerizable Compound (A)]

In a stereolithographic resin composition of the present invention, the polyfunctional (meth)acrylic polymerizable compound (A) is used to impart curability to the stereolithographic resin composition and strength and toughness to the cured product, in combination with the photopolymerization initiator (B).

Preferably, the polyfunctional (meth)acrylic polymerizable compound (A) comprises a polyfunctional (meth) acrylic polymerizable compound (A)-I having a molecular weight of less than 500 (also referred to simply as "polyfunctional (meth)acrylic polymerizable compound (A)-I"), and/or a polyfunctional (meth)acrylic polymerizable compound (A)-II having a molecular weight of 500 or more (also referred to simply as "polyfunctional (meth)acrylic polymerizable compound (A)-II"). The polyfunctional (meth)acrylic polymerizable compound (A)-I is used to impart especially strength to the cured product of the stereolithographic resin composition, whereas the polyfunctional (meth)acrylic polymerizable compound (A)-II is used to impart especially toughness to the cured product of the stereolithographic resin composition.

The polyfunctional (meth)acrylic polymerizable compound (A) may be used alone, or two or more thereof may be used in combination.

In view of superior water resistance, the polyfunctional (meth)acrylic polymerizable compound (A)-I having a molecular weight of less than 500 is preferably one that does not contain a polymer structure within a single molecule, more preferably a methacrylate that does not contain a polymer structure within a single molecule. The absence of a polymer structure leads to reduced content of repeating units of polar functional groups (ester groups, carbonate groups, urethane

groups, ether groups, amide groups, imide groups, and arylate groups) per molecule, which tends to improve water resistance.

Examples of the polymer structure include polyester, polycarbonate, polyurethane, polyether, polyamide, polyimide, polyarylate, and polyacrylate.

The polyfunctional (meth)acrylic polymerizable compound (A)-I requires a molecular weight of less than 500. In view of strength and toughness, the molecular weight is preferably 250 or more and 495 or less, more preferably 300 or more and 490 or less.

Examples of the polyfunctional (meth)acrylic polymerizable compound (A)-I include a polyfunctional (meth) acrylic polymerizable compound (A)-I-1 containing a urethane bond (hereinafter, also referred to simply as "polyfunctional (meth)acrylic polymerizable compound (A)-I-1"), and a polyfunctional (meth)acrylic polymerizable compound containing no urethane bond (A)-I-2 (hereinafter, also referred to simply as "polyfunctional (meth)acrylic polymerizable compound (A)-I-2"). In view of even superior toughness of the cured product and allowing the urethane bond to also serve as a hydrogen donating structure and achieve even greater curability and a further reduction of leachables from the cured product, it is preferable to comprise a polyfunctional (meth)acrylic polymerizable compound (A)-I-1. More preferably, the polyfunctional (meth)acrylic polymerizable compound (A)-I-1 is a (meth)acrylate that does not contain a polymer structure within a single molecule.

As an example, the polyfunctional (meth)acrylic polymerizable compound (A)-I-1 can be synthesized with ease by an addition reaction of an isocyanate group-containing compound having an alkylene backbone or phenylene backbone with a (meth)acrylic compound having a hydroxyl group (-OH).

The compound having an isocyanate group, and the (meth)acrylic compound having a hydroxyl group (-OH) may be compounds identical or similar to those exemplified below with respect to the synthesis of polyfunctional urethanized (meth)acrylic polymerizable compound (A)-II-1.

The addition reaction may be performed using known methods and conditions, and is not particularly limited.

Examples of the polyfunctional (meth)acrylic polymerizable compound (A)-I-1 include polyfunctional urethanized (meth)acrylates such as hexamethylenebis(2-carbamoyloxyethyl)dimethacrylate, 2,2,4-trimethylhexamethylenebis(2-carbamoyloxyethyl)dimethacrylate (commonly known as UDMA), 2,4-tolylenebis(2-carbamoyloxyethyl)di(meth) acrylate, bishydroxyethyl methacrylate-isophorone diurethane, and 2,4-tolylenebis(2-carbamoyloxyethyl)dimethacrylate. These may be used alone, or two or more thereof may be used in combination. In view of the strength and toughness of the shaped article, preferred among these are 2,2,4-trimethylhexamethylenebis(2-carbamoyloxyethyl)dimethacrylate, more preferably 2,2,4-trimethylhexamethylenebis(2-carbamoyloxyethyl)dimethacrylate, and bishydroxyethyl methacrylate-isophorone diurethane, even more preferably 2,2,4-trimethylhexamethylenebis(2-carbamoyloxyethyl)dimethacrylate.

In view of providing even superior strength and water resistance in the shaped article when combined with other components, the content of the polyfunctional (meth)acrylic polymerizable compound (A)-I is preferably 10 to 90 mass%, more preferably 20 to 80 mass%, even more preferably 25 to 70 mass%, particularly preferably 30 to 60 mass% in total 100 mass% of the polymerizable compounds.

The content of polyfunctional (meth)acrylic polymerizable compound (A)-I may be appropriately chosen from these ranges, and may be, for example, 25 to 55 mass% in total 100 mass% of the polymerizable compounds.

In another certain preferred embodiment, in view of even superior strength of the shaped article, the content of the polyfunctional (meth)acrylic polymerizable compound (A)-I is preferably 20 to 90 mass%, more preferably 25 to 80 mass%, even more preferably 30 to 70 mass%, particularly preferably 35 to 60 mass% in total 100 mass% of the polyfunctional (meth)acrylic polymerizable compound (A).

In yet another certain preferred embodiment, in view of providing even superior strength, toughness, and water resistance in the shaped article when combined with other components, the content of the polyfunctional (meth)acrylic polymerizable compound (A)-I is preferably 10 to 70 mass%, more preferably 20 to 65 mass%, even more preferably 20 to 60 mass%, particularly preferably 30 to 55 mass% in total 100 mass% of the stereolithographic resin composition. In view of enabling a further reduction of a decrease in toughness, the content of the polyfunctional (meth)acrylic polymerizable compound (A)-I is most preferably 35 to 50 mass%.

In any of the embodiments above, in view of enabling a further reduction of leachables from the cured product, the content of polyfunctional (meth)acrylic polymerizable compound (A)-I can be read as referring to the content of polyfunctional (meth)acrylic polymerizable compound (A)-I-1.

In another certain preferred embodiment, in view of providing even superior strength in the shaped article and allowing the urethane bond to also serve as a hydrogen donating structure and achieve even greater curability and a further reduction of leachables from the cured product, the content of the polyfunctional (meth)acrylic polymerizable compound (A)-I-1 in the polyfunctional (meth)acrylic polymerizable compound (A)-I is preferably 20 mass% or more, more preferably 40 mass% or more, even more preferably 60 mass% or more, particularly preferably 80 mass% or more in total 100 mass% of the polyfunctional (meth)acrylic polymerizable compound (A)-I.

In certain preferred embodiments, the content of the polyfunctional (meth)acrylic polymerizable compound

(A)-I-1 in the polyfunctional (meth)acrylic polymerizable compound (A)-I may be 100 mass%.

Examples of the polyfunctional (meth)acrylic polymerizable compound (A)-I include bifunctional (meth)acrylic polymerizable compounds, and tri- and higher-functional (meth)acrylic polymerizable compounds. However, in view of toughness of the cured product, preferred are bifunctional (meth)acrylic polymerizable compounds.

Examples of the polyfunctional (meth)acrylic polymerizable compound (A)-I-2 include 2,2-bis((meth)acryloyloxyphenyl)propane, 2,2-bis(4-(meth)acryloyloxyethoxyphenyl)propane, 2,2-bis(4-(meth)acryloyloxypropoxyphenyl)propane, 2-(4-(meth)acryloyloxydiethoxyphenyl)-2-(4-(meth)acryloyloxyethoxyphenyl)propane, 2,2-bis(4-(meth)acryloyloxyisopropoxyphenyl)propane, 2,2-bis(4-(meth)acryloyloxypolyethoxyphenyl)propane (with an average of 2.6 moles of ethoxy group added), 1,4-bis(2-(meth)acryloyloxyethyl)pyromellitate, glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,4-butanediol di(meth)acrylate, 1,6-hexanediol di(meth)acrylate, 2-ethyl-1,6-hexanediol di(meth)acrylate, 1,9-nonanediol di(meth)acrylate, 1,10-decanediol di(meth)acrylate, 1,2-bis(3-methacryloyloxy-2-hydroxypropoxy)ethane, and tricyclodecanedimethanol di(meth)acrylate.

Examples of the tri- and higher-functional polymerizable compounds include trimethylolpropane tri(meth)acrylate, trimethylolethane tri(meth)acrylate, trimethylol methane tri(meth)acrylate, pentaerythritol tri(meth)acrylate, and pentaerythritol tetra(meth)acrylate.

In a stereolithographic resin composition of the present invention, the polyfunctional (meth)acrylic polymerizable compound with a molecular weight of 500 or more (A)-II (hereinafter, also referred to simply as "polyfunctional (meth)acrylic polymerizable compound (A)-II") is used to impart toughness to the cured product.

The polyfunctional (meth)acrylic polymerizable compound (A)-II can be classified into a polyfunctional (meth)acrylic polymerizable compound that contains an oligomer or polymer structure, and a polyfunctional (meth)acrylic polymerizable compound that does not contain an oligomer or polymer structure. However, in view of providing superior strength and toughness in the shaped article when combined with other components, it is preferable to comprise a polyfunctional (meth)acrylic polymerizable compound that contains an oligomer or polymer structure.

The polyfunctional (meth)acrylic polymerizable compound (A)-II includes a polyfunctional (meth)acrylic polymerizable compound (A)-II-1 containing a urethane bond (hereinafter, also referred to simply as "polyfunctional (meth)acrylic polymerizable compound (A)-II-1"), and a polyfunctional (meth)acrylic polymerizable compound containing no urethane bond (A)-II-2. However, in view of superior toughness of the cured product and allowing the urethane bond to also serve as a hydrogen donating structure and achieve even greater curability and a further reduction of leachables from the cured product, it is preferable to comprise a polyfunctional (meth)acrylic polymerizable compound (A)-II-1, more preferably a polyfunctional urethanized (meth)acrylic polymerizable compound (A)-II-1 that contains an oligomer or polymer structure.

In view of even superior toughness of the cured product and allowing the urethane bond to also serve as a hydrogen donating structure and achieve even greater curability and a further reduction of leachables from the cured product, a certain preferred embodiment is, for example, a stereolithographic resin composition that comprises a polyfunctional (meth)acrylic polymerizable compound (A)-I-1, and/or a polyfunctional (meth)acrylic polymerizable compound (A)-II-1.

In view of superior strength and water resistance of the cured product and providing even greater toughness in the cured product while reducing leachables from the cured product, a particularly preferred embodiment is, for example, a stereolithographic resin composition that comprises a polyfunctional (meth)acrylic polymerizable compound (A)-I-1, and a polyfunctional (meth)acrylic polymerizable compound (A)-II-1.

When the polyfunctional (meth)acrylic polymerizable compound (A)-II-1 contains an oligomer or polymer structure, the polyfunctional (meth)acrylic polymerizable compound (A)-II-1 containing an oligomer or polymer structure can be easily synthesized through an addition reaction between a polyol having a polymer backbone (e.g., a polymer structure such as a polyester, a polycarbonate, a polyurethane, a polyether, a polydiene, and a hydrogenated polydiene), a compound having an isocyanate group (-NCO), and a (meth)acrylic compound having a hydroxyl group (-OH). The polyfunctional (meth)acrylic polymerizable compound (A)-II-1 containing an oligomer or polymer structure can also be easily synthesized by allowing lactone or alkylene oxide to undergo a ring-opening addition reaction with a (meth)acrylic compound having a hydroxyl group, and causing the resulting compound with a terminal hydroxyl group to undergo an addition reaction with a compound having an isocyanate group.

When the polyfunctional (meth)acrylic polymerizable compound (A)-II-1 contains an oligomer or polymer structure, the polyfunctional (meth)acrylic polymerizable compound (A)-II-1 containing an oligomer or polymer structure is preferably a (meth)acrylate that comprises, within a single molecule, at least one structure selected from the group consisting of a polyester, a polycarbonate, a polyurethane, a polyether, a poly-conjugated diene, and a hydrogenated poly-conjugated diene.

In these structures, examples of the polyester include copolymers of dicarboxylic acids (for example, aromatic dicarboxylic acids such as phthalic acid and isophthalic acid, and unsaturated aliphatic dicarboxylic acids such as maleic

acid) and aliphatic diols having 2 to 18 carbon atoms, copolymers of dicarboxylic acids (for example, saturated aliphatic dicarboxylic acids such as adipic acid and sebacic acid) and aliphatic diols having 2 to 18 carbon atoms, β-propiolactone polymers, γ-butyrolactone polymers, δ-valerolactone polymers, ε-caprolactone polymers, and copolymers of these. Preferred are copolymers of dicarboxylic acids (preferably, aromatic dicarboxylic acids, and unsaturated aliphatic dicarboxylic acids) and aliphatic diols having 2 to 12 carbon atoms, and copolymers of dicarboxylic acids (preferably, saturated aliphatic dicarboxylic acids) and aliphatic glycols having 2 to 12 carbon atoms.

Examples of the polycarbonate include polycarbonates derived from C2 to C18 aliphatic diols, polycarbonates derived from bisphenol A, and polycarbonates derived from C2 to C18 aliphatic diols and bisphenol A. Preferred are polycarbonates derived from C2 to C12 aliphatic diols, polycarbonates derived from bisphenol A, and polycarbonates derived from C2 to C12 aliphatic diols and bisphenol A.

Examples of the polyurethane include polymers of C2 to C18 aliphatic diols and C1 to C18 diisocyanates. Preferred are polymers of C2 to C12 aliphatic diols and C1 to C12 diisocyanates.

Examples of the polyether include polyethylene glycol, polypropylene glycol, polybutylene glycol, and poly(1-methylbutylene glycol).

Examples of the poly-conjugated diene and hydrogenated poly-conjugated diene include 1,4-polybutadiene, 1,2-polybutadiene, polyisoprene, poly(butadiene-isoprene), poly(butadiene-styrene), poly(isoprene-styrene), polyfarnesene, and hydrogenated products of these. In view of superior toughness and water resistance, preferred among these are the polyester structures.

In view of even superior toughness and water resistance, the polyfunctional (meth)acrylic polymerizable compound (A)-II-1 is preferably a (meth)acrylate that comprises, within a single molecule, at least one polyol moiety selected from the group consisting of a polyester, a polycarbonate, a polyurethane, a polyether, a poly-conjugated diene, and a hydrogenated poly-conjugated diene each having a structure derived from a C4 to C18 aliphatic chain diol unit having a branched structure.

Examples of the polyester include copolymers with a structure derived from a C4 to C18 aliphatic chain diol unit having a branched structure, and with a structure derived from a C4 to C18 aliphatic chain dicarboxylic acid and/or aromatic dicarboxylic acid unit having no branched structure.

Examples of the polycarbonate include copolymers with a structure derived from a C4 to C18 aliphatic chain diol unit having a branched structure, and with a structure derived from a C4 to C18 aliphatic chain diol unit having no branched structure.

Examples of the polyurethane include structures derived from a C4 to C18 aliphatic chain diol unit having a branched structure, and polycondensates of diisocyanate compounds.

Examples of the polyether include polyethers with a structure derived from a C4 to C18 aliphatic chain diol unit having a branched structure, and polyethers with a structure derived from a C4 to C18 aliphatic chain diol unit having no branched structure.

Examples of the poly-conjugated diene include homopolymers or copolymers of conjugated diene polymerizable compounds. Examples of the conjugated diene polymerizable compounds include 1,3-butadiene, isoprene, 2,3-dimethyl-1,3-butadiene, 1,3-pentadiene, and 1,3-hexadiene.

Examples of the hydrogenated poly-conjugated diene include hydrogenated polybutadiene, hydrogenated polyisoprene, and hydrogenated polyisobutylene.

Among these, in view of superior toughness and water resistance, it is preferable to comprise, as a polymer backbone, at least one structure selected from the group consisting of a polyester, a polycarbonate, a polyether, and a hydrogenated poly-conjugated diene, more preferably at least one structure selected from the group consisting of a polyester and a polycarbonate, even more preferably at least one structure selected from the group consisting of polyesters.

Examples of the diols constituting the C4 to C18 aliphatic chain diol unit having a branched structure include 2-methyl-1,3-propanediol, 2,2-diethyl-1,3-propanediol, 1,3-butanediol, 2-methyl-1,4-butanediol, neopentyl glycol, 3-methyl-1,5-pentanediol, 2-methyl-1,8-octanediol, 2,7-dimethyl-1,8-octanediol, 2-methyl-1,9-nonanediol, 2,8-dimethyl-1,9-nonanediol, 2-methyl-1,10-decanediol, 2,9-dimethyl-1,10-decanediol, 2-methyl-1,11-undecanediol, 2,10-dimethyl-1,11-undecanediol, 2-methyl-1,12-dodecanediol, 2,11-dimethyl-1,12-dodecanediol, 2-methyl-1,13-tridecanediol, 2,12-dimethyl-1,13-tridecanediol, 2-methyl-1,14-tetradecanediol, 2,13-dimethyl-1,14-tetradecanediol, 2-methyl-1,15-pentadecanediol, 2,14-dimethyl-1,15-pentadecanediol, 2-methyl-1,16-hexadecanediol, and 2,15-dimethyl-1,16-hexadecanediol. In view of providing a stereolithographic resin composition having superior curability and low viscosity, preferred for use as polyols are C5 to C12 aliphatic diols having a methyl-group side chain, such as 2-methyl-1,4-butanediol, 3-methyl-1,5-pentanediol, 2-methyl-1,8-octanediol, 2,7-dimethyl-1,8-octanediol, 2-methyl-1,9-nonanediol, and 2,8-dimethyl-1,9-nonanediol, more preferably 2-methyl-1,4-butanediol, 3-methyl-1,5-pentanediol, 2-methyl-1,8-octanediol, and 2,7-dimethyl-1,8-octanediol, even more preferably 3-methyl-1,5-pentanediol, and 2-methyl-1,8-octanediol.

Examples of the compound having an isocyanate group include hexamethylene diisocyanate (HDI), tolylene

diisocyanate (TDI), xylylene diisocyanate (XDI), diphenylmethane diisocyanate (MDI), isophorone diisocyanate (IPDI), trimethylhexamethylene diisocyanate (TMHMDI), tricyclodecane diisocyanate (TCDDI), and adamantane diisocyanate (ADI).

Examples of the (meth)acrylic compound having a hydroxyl group include hydroxy (meth)acrylate compounds such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-bis[4-(2-hydroxy-3-(meth)acryloyloxypropoxy)phenyl]propane, 1,2-bis[3-(meth)acryloyloxy-2-hydroxypropoxy]ethane, pentaerythritol tri(meth)acrylate, and tri or tetra(meth)acrylates of dipentaerythritol; and hydroxy(meth)acrylamide compounds such as N-hydroxyethyl(meth)acrylamide, and N,N-bis(2-hydroxyethyl)(meth)acrylamide.

When the desired polyfunctional (meth)acrylic polymerizable compound (A)-II-1 is a (meth)acrylate compound, it can be produced by selecting a hydroxy(meth)acrylate compound.

The addition reaction between a compound having an isocyanate group and a (meth)acrylic compound having a hydroxyl group may be performed using known methods and conditions, and is not particularly limited.

Examples of the polyfunctional urethanized (meth)acrylic polymerizable compound (A)-II-1 with no polymer structure include dipentaerythritol penta(meth)acrylate hexamethylene diisocyanate urethane prepolymer.

The polyfunctional (meth)acrylic polymerizable compound (A)-II-1 requires a molecular weight of 500 or more. The molecular weight of polyfunctional (meth)acrylic polymerizable compound (A)-II-1 is preferably 600 or more, more preferably 700 or more. In view of strength and toughness, the molecular weight is even more preferably 750 or more, particularly preferably 1,000 or more. The molecular weight of polyfunctional (meth)acrylic polymerizable compound (A)-II-1 is preferably 6,000 or less, more preferably 5,500 or less. In view of strength and toughness, the molecular weight is even more preferably 5,000 or less, particularly preferably 3,000 or less.

Taken together, in view of strength and toughness, the preferred molecular weight of polyfunctional (meth)acrylic polymerizable compound (A)-II-1 is 750 to 5,000, more preferably 1,000 to 3,000.

Examples of the polyfunctional (meth)acrylic polymerizable compound (A)-II include bifunctional (meth)acrylic polymerizable compounds, and tri- and higher-functional (meth)acrylic polymerizable compounds.

In a certain preferred embodiment, in view of even superior strength, toughness, and water resistance of the shaped article and allowing the urethane bond to also serve as a hydrogen donating structure and achieve even greater curability and a further reduction of leachables from the cured product when combined with other components, the content of polyfunctional (meth)acrylic polymerizable compound (A)-II-1 is preferably 5 to 80 mass%, more preferably 10 to 70 mass%, even more preferably 15 to 60 mass%, particularly preferably 20 to 50 mass% in total 100 mass% of the polymerizable compounds.

In another certain preferred embodiment, in view of even superior strength, toughness, and water resistance of the shaped article and allowing the urethane bond to also serve as a hydrogen donating structure and achieve even greater curability and a further reduction of leachables from the cured product when combined with other components, the content of polyfunctional (meth)acrylic polymerizable compound (A)-II-1 is preferably 10 to 75 mass%, more preferably 15 to 70 mass%, even more preferably 20 to 65 mass%, particularly preferably 25 to 60 mass% in total 100 mass% of the polyfunctional (meth)acrylic polymerizable compound (A).

In yet another certain preferred embodiment, in view of even superior strength, toughness, and water resistance of the shaped article and allowing the urethane bond to also serve as a hydrogen donating structure and achieve even greater curability and a further reduction of leachables from the cured product when combined with other components, the content of polyfunctional (meth)acrylic polymerizable compound (A)-II-1 is preferably 1 to 70 mass%, more preferably 5 to 60 mass%, even more preferably 10 to 50 mass%, particularly preferably 20 to 40 mass% in total 100 mass% of the stereolithographic resin composition.

In view of even superior strength, toughness, and water resistance of the cured product and allowing the urethane bond to also serve as a hydrogen donating structure and achieve even greater curability and a further reduction of leachables from the cured product when combined with other components, the content of polyfunctional (meth)acrylic polymerizable compound (A)-II-1 is preferably 20 mass% or more, more preferably 40 mass% or more, even more preferably 60 mass% or more, particularly preferably 80 mass% or more in total 100 mass% of the polyfunctional (meth) acrylic polymerizable compound (A)-II.

The content of the polyfunctional (meth)acrylic polymerizable compound (A)-II-1 in the polyfunctional (meth) acrylic polymerizable compound (A)-II may be 100 mass%.

Examples of the polyfunctional (meth)acrylic polymerizable compound (A)-II-2 containing no urethane bond include bifunctional (meth)acrylic polymerizable compounds, and tri- and higher-functional (meth)acrylic polymerizable compounds.

Examples of the polyfunctional (meth)acrylic polymerizable compound (A)-II-2 containing no urethane bond include bifunctional (meth)acrylic polymerizable compounds such as 2,2-bis(4-(meth)acryloyloxydiethoxyphenyl)propane, 2,2-bis[4-(2-hydroxy-3-methacryloyloxypropoxy)phenyl]propane (commonly known as Bis-GMA), 2,2-

bis(4-(meth)acryloyloxytetraethoxyphenyl)propane, 2,2-bis(4-(meth)acryloyloxypentaethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypolyethoxyphenyl)propane (for example, with an average of 4 or more moles of ethoxy group added), and polyethylene glycol di(meth)acrylate; and tri- and higher-functional (meth)acrylic polymerizable compounds such as dipentaerythritol penta(meth)acrylate, and 1,7-di(meth)acryloyloxy-2,2,6,6-tetra(meth)acryloyloxymethyl-4-oxaheptane.

In view of even superior water resistance, the polyfunctional (meth)acrylic polymerizable compound (A)-II-2 containing no urethane bond is preferably a polymerizable compound with no hydroxyl group, for example.

In view of providing even superior strength, toughness, and water resistance in the cured product when combined with other components, the content of the polyfunctional (meth)acrylic polymerizable compound (A)-II in a stereolithographic resin composition of the present invention is preferably 1 to 80 mass%, more preferably 5 to 70 mass%, even more preferably 10 to 60 mass%, particularly preferably 20 to 50 mass% in total 100 mass% of the polymerizable compounds.

The content of the polyfunctional (meth)acrylic polymerizable compound (A)-II may be appropriately chosen from these ranges, and may be, for example, 15 to 45 mass% in total 100 mass% of the polymerizable compounds.

The polyfunctional (meth)acrylic polymerizable compound (A)-II may be used alone, or two or more thereof may be used in combination.

In view of providing even superior strength, toughness, and water resistance in the cured product when combined with other components, the content of the polyfunctional (meth)acrylic polymerizable compound (A)-II in a stereolithographic resin composition of the present invention is preferably 10 to 60 mass%, more preferably 15 to 65 mass%, even more preferably 20 to 50 mass%, particularly preferably 25 to 45 mass%, most preferably 25 to 40 mass% in total 100 mass% of the stereolithographic resin composition.

In view of even superior strength, toughness, and water resistance of the cured product, the ratio (A)-I:(A)-II of the content of the polyfunctional (meth)acrylic polymerizable compound (A)-I and the content of the polyfunctional (meth) acrylic polymerizable compound (A)-II in a stereolithographic resin composition of the present invention is preferably 5:95 to 95:5, more preferably 10:90 to 90:10, even more preferably 20:80 to 80:20.

In view of providing even superior strength, toughness, and water resistance in the cured product, a certain preferred embodiment is, for example, a stereolithographic resin composition in which the polyfunctional (meth)acrylic polymerizable compound (A) comprises a polyfunctional (meth)acrylic polymerizable compound (A)-I having a molecular weight of less than 500, and a polyfunctional (meth)acrylic polymerizable compound (A)-II having a molecular weight of 500 or more, and the mass ratio (A)-I:(A)-II of the content of the polyfunctional (meth)acrylic polymerizable compound (A)-I and the content of the polyfunctional (meth)acrylic polymerizable compound (A)-II is 51:49 to 95:5. The mass ratio (A)-I:(A)-II is more preferably 52:48 to 90:10, even more preferably 55:45 to 80:20.

In such a preferred embodiment of the stereolithographic resin composition, the content of the monofunctional (meth)acrylic polymerizable compound (C) is, for example, 7 to 40 mass% in total 100 mass% of the polymerizable compounds.

The content of the polyfunctional (meth)acrylic polymerizable compound (A) in a stereolithographic resin composition of the present invention is preferably 10 to 95 mass% in total 100 mass% of the polymerizable compounds. In view of providing even superior strength, toughness, and water resistance in the shaped article when combined with other components, the content of the polyfunctional (meth)acrylic polymerizable compound (A) is more preferably 20 to 92 mass%, even more preferably 30 to 90 mass%, particularly preferably 40 to 85 mass%.

In view of providing even superior strength, toughness, and water resistance in the shaped article when combined with other components, the content of the polyfunctional (meth)acrylic polymerizable compound (A) in a stereolithographic resin composition of the present invention is preferably 10 to 96 mass%, more preferably 20 to 94 mass%, even more preferably 30 to 92 mass%, particularly preferably 40 to 90 mass%, most preferably 50 to 90 mass% in total 100 mass% of the stereolithographic resin composition.

[Photopolymerization Initiator Containing Four or More Heteroatoms within a Single Molecule (B)]

In a stereolithographic resin composition of the present invention, the photopolymerization initiator containing four or more heteroatoms within a single molecule (B) (also referred to simply as "photopolymerization initiator (B)") used in the present invention is used to impart curability to the stereolithographic resin composition and low leachability to the cured product, in combination with the polyfunctional (meth)acrylic polymerizable compound (A), more preferably with optional addition of monofunctional (meth)acrylic polymerizable compound (C).

It is important that the photopolymerization initiator (B) of the present invention comprise four or more heteroatoms within a single molecule.

The inclusion of four or more heteroatoms within a single molecule increases the absorption coefficient, increasing the number of cleavage sites and providing an increased number of active species generated per molecule for the initiation of polymerization. This leads to improved initiator efficiency (the proportion of the photopolymerization

initiator that actually functions as an initiator), enabling polymerization initiation even with a low irradiation dose.

Presumably, the active species produced by cleavage upon photoirradiation for the initiation of polymerization preferentially participate in the polymerization initiation reaction rather than in the concomitant hydrogen abstraction reaction.

Residues generated from the photopolymerization initiator in a hydrogen abstraction reaction are converted into free low-molecular compounds, leading to an increased amount of leachables. In contrast, in polymerize initiation reactions, there is no leaching because the photopolymerization initiator becomes incorporated into the cured product in the form of residues.

On this basis, the photopolymerization initiator (B) of the present invention, when combined with the polyfunctional (meth)acrylic polymerizable compound (A), can improve the curability of the stereolithographic resin composition and enhance the mechanical properties of the cured product, thereby achieving excellent shape accuracy, strength, toughness, and water resistance while reducing leachables from the shaped article (cured product).

The heteroatoms contained in the photopolymerization initiator (B) of the present invention are any atoms other than carbon and hydrogen, and, in view of more effectively providing a longer lifetime for the active species generated from the photopolymerization initiator for the initiation of polymerization and thus achieving favorable initiator efficiency, the photopolymerization initiator (B) comprises preferably nitrogen atoms, oxygen atoms, fluorine atoms, phosphorus atoms, sulfur atoms, iron atoms, or titanium atoms, more preferably at least one selected from the group consisting of a nitrogen atom, an oxygen atom, a phosphorus atom, and a sulfur atom. In view of reducing the likelihood of photoabsorption in the visible light region and reducing the risk of impairing color tone, it is even more preferable to comprise at least one selected from an oxygen atom and a phosphorus atom.

The photopolymerization initiator (B) may comprise only one kind of heteroatom, or two or more kinds of heteroatoms.

In view of providing superior shape accuracy, strength, toughness, and water resistance while enabling a reduction of leachables from the cured product when combined with the polyfunctional (meth)acrylic polymerizable compound (A), the photopolymerization initiator (B) of the present invention has a molecular weight of preferably 400 or more. By having a molecular weight of 400 or more, namely an increased molecular size, the photopolymerization initiator (B) exhibits reduced mobility within the cured product, enabling a reduction of leachables from the cured product of a stereolithographic resin composition of the present invention.

A certain preferred embodiment is, for example, a stereolithographic resin composition in which at least one of the heteroatoms in the photopolymerization initiator (B) is one selected from the group consisting of an oxygen atom and a phosphorus atom, and the molecular weight of the photopolymerization initiator (B) is 400 or more.

In view of improving the curability of the stereolithographic resin composition and enhancing the mechanical properties of the cured product and thereby achieving excellent shape accuracy, strength, toughness, and water resistance while enabling a further reduction of leachables from the cured product, it is preferable that the photopolymerization initiator (B) of the present invention comprise two or more carbonyl groups within a single molecule. The inclusion of two or more carbonyl groups within a single molecule increases the number of cleavage sites, providing an increased number of active species generated per molecule for the initiation of polymerization. This leads to even greater initiator efficiency, enabling polymerization initiation even with a low irradiation dose when combined with the polyfunctional (meth) acrylic polymerizable compound (A) within the stereolithographic resin composition. This allows the cured product of a stereolithographic resin composition of the present invention to achieve a high polymerization conversion rate, leading to excellent mechanical properties and a dense structure, thereby reducing leaching from the cured product.

A certain preferred embodiment is, for example, a stereolithographic resin composition in which the photopolymerization initiator (B) comprises two or more carbonyl groups within a single molecule, and has a molecular weight of 400 or more.

Another certain preferred embodiment is, for example, a stereolithographic resin composition in which the heteroatoms of the photopolymerization initiator (B) are oxygen atoms and phosphorus atoms, and the photopolymerization initiator (B) comprises two or more carbonyl groups within a single molecule, and has a molecular weight of 400 or more.

Examples of the photopolymerization initiator (B) include (bis)acylphosphine oxides, thioxanthones or quaternary ammonium salts of thioxanthones, ketals, $\alpha$-diketones, coumarins, anthraquinones, benzoin alkyl ether compounds, and $\alpha$-aminoketone compounds. The photopolymerization initiator (B) may be used alone, or two or more thereof may be used in combination.

Preferred among such photopolymerization initiators (B) is at least one selected from the group consisting of bisacylphosphine oxides and oxime esters. More preferred are bisacylphosphine oxides. In this way, a stereolithographic resin composition can be obtained that has excellent photocurability both in the ultraviolet and visible regions, and that shows sufficient photocurability regardless of whether the light source used is a laser, a halogen lamp, a light emitting diode (LED), or a xenon lamp.

Examples of bisacylphosphine oxides include bis(2,6-dichlorobenzoyl)phenylphosphine oxide, bis(2,6-dichlor-

obenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, bis(2,5,6-trimethylbenzoyl)-2,4,4-trimethylpentylphosphine oxide, and ethyl phenyl(2,4,6-trimethylbenzoyl)phosphinate. Other examples include the compounds mentioned in JP2000-159621 A. In view of an easier reduction of leachables from the cured product through integrated action with the polyfunctional (meth)acrylic polymerizable compound (A) in the stereolithographic resin composition, preferred among these are bis(2,6-dichlorobenzoyl)phenylphosphine oxide, bis(2,6-dichlorobenzoyl)-2,5-dimethylphenylphosphine oxide, bis(2,6-dimethoxybenzoyl)phenylphosphine oxide, bis(2,6-dimethoxybenzoyl)-2,5-dimethylphenylphosphine oxide, bis(2,4,6-trimethylbenzoyl)phenylphosphine oxide, and ethyl phenyl(2,4,6-trimethylbenzoyl)phosphinate, more preferably bis(2,6-dimethoxybenzoyl)phenylphosphine oxide, bis(2,6-dimethoxybenzoyl)-2,5-dimethylphenylphosphine oxide, and bis(2,4,6-trimethylbenzoyl)phenylphosphine oxide, even more preferably bis(2,4,6-trimethylbenzoyl)phenylphosphine oxide.

Examples of oxime ester compounds that can be used as the photopolymerization initiator include 1,2-octanedione 1-[4-(phenylthio)phenyl]-2-(o-benzoyloxime) (another name: 1-[4-(phenylthio)phenyl]octane-1,2-dione-2-(O-benzoyloxime)), and ethanone, 1-[9-ethyl-6-(2-methylbenzoyl)-9H-carbazol-3-yl]-, 1-(o-acetyloxime) (another name: Irgacure OXE-02).

In view of shape accuracy, it is important that the content of the photopolymerization initiator (B) in a stereolithographic resin composition of the present invention is 0.75 to 3.0 parts by mass relative to total 100 parts by mass of the polymerizable compounds.

When the content of photopolymerization initiator (B) is less than 0.75 parts by mass, it results in increased transmission of irradiation light through the resin composition, leading to a decrease in shape accuracy and possibly preventing the formation of a shaped article with the desired shape.

On the other hand, when the content of photopolymerization initiator (B) exceeds 3.0 parts by mass, it leads to greater shielding of light, resulting in weak bonding between layers and possible molding defects.

In view of providing superior shape accuracy, strength, toughness, and water resistance while achieving a further reduction of leachables from the cured product through integrated action with the polyfunctional (meth)acrylic polymerizable compound (A) (optionally with the monofunctional (meth)acrylic polymerizable compound (C)), the content of photopolymerization initiator (B) is more preferably 0.8 parts or more by mass, even more preferably 0.9 parts or more by mass, particularly preferably 1.0 part or more by mass relative to total 100 parts by mass of the polymerizable compounds.

In view of providing superior shape accuracy, strength, toughness, and water resistance while achieving a further reduction of leachables from the cured product through integrated action with the polyfunctional (meth)acrylic polymerizable compound (A) (optionally with the monofunctional (meth)acrylic polymerizable compound (C)), the content of photopolymerization initiator (B) is more preferably 2.5 parts or less by mass, even more preferably 2.0 parts or less by mass, particularly preferably 1.5 parts or less by mass relative to total 100 parts by mass of the polymerizable compounds.

A stereolithographic resin composition of the present invention is not particularly limited, and may comprise other components for example, as long as it comprises the polyfunctional (meth)acrylic polymerizable compound (A) and photopolymerization initiator (B). The method of production of a stereolithographic resin composition of the present invention is not particularly limited, and a stereolithographic resin composition of the present invention can be produced following known methods of producing stereolithographic resin compositions.

[Monofunctional (Meth)Acrylic Polymerizable Compound (C)]

Preferably, a stereolithographic resin composition of the present invention further comprises a monofunctional (meth)acrylic polymerizable compound (C) for the purpose of reducing viscosity to achieve improved shapeability and improving toughness and water resistance through integrated action with the polyfunctional (meth)acrylic polymerizable compound (A).

The monofunctional (meth)acrylic polymerizable compound (C) may be used alone, or two or more thereof may be used in combination.

A certain preferred embodiment is, for example, a stereolithographic resin composition in which the monofunctional (meth)acrylic polymerizable compound (C) is a monofunctional acrylic polymerizable compound.

Examples of the monofunctional (meth)acrylic polymerizable compound (C) include (meth)acrylic acid ester compounds containing two or more aromatic rings, such as o-phenylphenyl (meth)acrylate, m-phenylphenyl (meth)acrylate, p-phenylphenyl (meth)acrylate, ethoxylated o-phenylphenol (meth)acrylate, ethoxylated m-phenylphenol (meth)acrylate, ethoxylated p-phenylphenol (meth)acrylate, propoxylated o-phenylphenol (meth)acrylate, propoxylated m-phenylphenol (meth)acrylate, propoxylated p-phenylphenol (meth)acrylate, butoxylated o-phenylphenol (meth)acry-

late, butoxylated m-phenylphenol (meth)acrylate, butoxylated p-phenylphenol (meth)acrylate, diphenylmethyl (meth)acrylate, 4-(1-methyl-1-phenylethyl)(meth)acrylate, triphenylmethyl (meth)acrylate, o-phenoxyphenyl (meth)acrylate, m-phenoxyphenyl (meth)acrylate, p-phenoxyphenyl (meth)acrylate, o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, 2-(p-phenoxyphenyl)ethyl (meth)acrylate, 3-(o-phenoxyphenyl)propyl (meth)acrylate, 3-(m-phenoxyphenyl)propyl (meth)acrylate, 3-(p-phenoxyphenyl)propyl (meth)acrylate, 9-(meth)acryloyloxyfluorene, 9-(meth)acryloyloxymethylfluorene, N-(meth)acryloylcarbazole, and N-(meth)acryloylmethylcarbazole; (meth)acrylic acid ester compounds containing a single aromatic ring, such as phenyl (meth)acrylate, benzyl (meth)acrylate, phenoxyethyl (meth)acrylate, phenoxybutyl (meth)acrylate, phenoxydiethylene glycol (meth)acrylate, phenoxypolyethylene glycol (meth)acrylate, 4-methylphenyl (meth)acrylate, 4-n-butylphenyl (meth)acrylate, 4-t-butylphenyl (meth)acrylate, 4-nonylphenyl (meth)acrylate, o-2-propenylphenyl (meth)acrylate, benzhydrol (meth)acrylate, and cumylphenol (meth)acrylate; alicyclic (meth)acrylic acid ester compounds such as cyclic trimethylolpropane formal (meth)acrylate, isobornyl (meth)acrylate, 2-(1-adamantyl)propyl (meth)acrylate, 2-methyladamantan-2-yl (meth)acrylate, 2-ethyladamantan-2-yl (meth)acrylate, 2-n-propyladamantan-2-yl (meth)acrylate, 2-isopropyladamantan-2-yl (meth)acrylate, 1-(adamantan-1-yl)-1-methylethyl (meth)acrylate, 1-(adamantan-1-yl)-1-ethylethyl (meth)acrylate, 1-(adamantan-1-yl)-1-methylpropyl (meth)acrylate, and 1-(adamantan-1-yl)-1-ethylpropyl (meth)acrylate; nitrogen atom-containing cyclic (meth)acrylic acid ester compounds such as pentamethylpiperidinyl (meth)acrylate, tetramethylpiperidinyl (meth)acrylate, and 4-(pyrimidin-2-yl)piperazin-1-yl (meth)acrylate; and cyclic (meth)acrylamide compounds such as N-(meth)acryloylmorpholine, N-(meth)acryloylpyrrolidine, N-(meth)acryloylpiperidine, N-(meth)acryloyl-2-methylpiperidine, and N-(meth)acryloyl-2,2,6,6-tetramethylpiperidine.

Preferred among these are (meth)acrylic acid ester compounds containing two or more aromatic rings, and alicyclic (meth)acrylic acid ester compounds. In view of reducing viscosity and providing excellent shapeability in the stereolithographic resin composition and achieving even superior water resistance in the shaped article, more preferred are (meth)acrylic acid ester compounds containing two or more aromatic rings, even more preferably ethoxylated o-phenylphenol (meth)acrylate, ethoxylated m-phenylphenol (meth)acrylate, ethoxylated p-phenylphenol (meth)acrylate, o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, and 2-(p-phenoxyphenyl)ethyl (meth)acrylate, yet more preferably ethoxylated o-phenylphenol (meth)acrylate, ethoxylated m-phenylphenol (meth)acrylate, ethoxylated p-phenylphenol (meth)acrylate, o-phenoxybenzyl (meth)acrylate, m-phenoxybenzyl (meth)acrylate, and p-phenoxybenzyl (meth)acrylate, particularly preferably ethoxylated o-phenylphenol (meth)acrylate, o-phenoxybenzyl (meth)acrylate, and m-phenoxybenzyl (meth)acrylate, most preferably m-phenoxybenzyl (meth)acrylate.

The content of the monofunctional (meth)acrylic polymerizable compound (C) in a stereolithographic resin composition of the present invention is preferably 1.0 to 60 mass% in total 100 mass% of the polymerizable compounds. In view of shape accuracy and even superior toughness and water resistance of the cured product, the content of monofunctional (meth)acrylic polymerizable compound (C) is more preferably 2.5 to 50 mass%, even more preferably 5 to 45 mass%, particularly preferably 7 to 40 mass%.

In a certain preferred embodiment, in view of providing even superior strength, toughness, and water resistance in the shaped article when combined with other components, the content of monofunctional (meth)acrylic polymerizable compound (C) is preferably 1.0 to 60 mass%, more preferably 2.5 to 50 mass%, even more preferably 5 to 45 mass%, particularly preferably 7 to 40 mass% in total 100 mass% of the stereolithographic resin composition.

A stereolithographic resin composition of the present invention may additionally comprise polymerizable compounds other than the polyfunctional (meth)acrylic polymerizable compound (A) and monofunctional (meth)acrylic polymerizable compound (C) (hereinafter, such polymerizable compounds will also be referred to simply as "additional polymerizable compounds").

A certain preferred embodiment is, for example, a stereolithographic resin composition in which the polymerizable compounds consist essentially of polyfunctional (meth)acrylic polymerizable compound (A) and monofunctional (meth)acrylic polymerizable compound (C).

Concerning the phrase "polymerizable compounds consisting essentially of polyfunctional (meth)acrylic polymerizable compound (A) and monofunctional (meth)acrylic polymerizable compound (C)", the content of polymerizable compounds other than the polyfunctional (meth)acrylic polymerizable compound (A) and monofunctional (meth)acrylic polymerizable compound (C) is preferably less than 10 mass%, more preferably less than 5 mass%, even more preferably less than 1 mass%, particularly preferably less than 0.1 mass% in total 100 mass% of the stereolithographic resin composition.

Examples of the additional polymerizable compounds include (meth)acrylamide oligomers (for example, those with a weight-average molecular weight of 1,000 or less, or those with a weight-average molecular weight of 1,000 or more).

In a certain preferred embodiment, a stereolithographic resin composition is essentially free of (meth)acrylamide oligomers (for example, those with a molecular weight of 1,000 or less, or those with a molecular weight of 1,000 or more).

Here, by "essentially free of (meth)acrylamide oligomers", it means that the content of (meth)acrylamide oligomers is less than 5 mass%, preferably less than 1 mass%, more preferably less than 0.1 mass%, even more preferably less than 0.01 mass% in total 100 mass% of the stereolithographic resin composition.

Concerning the content of additional polymerizable compounds, the phrase "essentially free of additional polymerizable compounds" is intended to have the same meaning as the phrase "essentially free of (meth)acrylamide oligomers."

A certain preferred embodiment is, for example, a stereolithographic resin composition in which the polyfunctional (meth)acrylic polymerizable compound (A) comprises a polyfunctional (meth)acrylic polymerizable compound (A)-I having a molecular weight of less than 500, and the photopolymerization initiator (B) has a molecular weight of 400 or more.

Another certain preferred embodiment is, for example, a stereolithographic resin composition in which the polyfunctional (meth)acrylic polymerizable compound (A) comprises a polyfunctional (meth)acrylic polymerizable compound (A)-I having a molecular weight of less than 500, and the photopolymerization initiator (B) comprises two or more carbonyl groups within a single molecule.

Yet another certain preferred embodiment is, for example, a stereolithographic resin composition in which the polyfunctional (meth)acrylic polymerizable compound (A) comprises a polyfunctional (meth)acrylic polymerizable compound (A)-II having a molecular weight of 500 or more, and the photopolymerization initiator (B) has a molecular weight of 400 or more.

Still another certain preferred embodiment is, for example, a stereolithographic resin composition in which the polyfunctional (meth)acrylic polymerizable compound (A) comprises a polyfunctional (meth)acrylic polymerizable compound (A)-II having a molecular weight of 500 or more, and the photopolymerization initiator (B) comprises two or more carbonyl groups within a single molecule.

Yet another certain preferred embodiment is, for example, a stereolithographic resin composition in which the polyfunctional (meth)acrylic polymerizable compound (A) comprises a polyfunctional (meth)acrylic polymerizable compound (A)-I having a molecular weight of less than 500 and a polyfunctional (meth)acrylic polymerizable compound (A)-II having a molecular weight of 500 or more, and the photopolymerization initiator (B) has a molecular weight of 400 or more.

Still another certain preferred embodiment is, for example, a stereolithographic resin composition in which the polyfunctional (meth)acrylic polymerizable compound (A) comprises a polyfunctional (meth)acrylic polymerizable compound (A)-I having a molecular weight of less than 500 and a polyfunctional (meth)acrylic polymerizable compound (A)-II having a molecular weight of 500 or more, and the photopolymerization initiator (B) comprises two or more carbonyl groups within a single molecule.

Yet another certain preferred embodiment is, for example, a stereolithographic resin composition in which the polyfunctional (meth)acrylic polymerizable compound (A) comprises a polyfunctional (meth)acrylic polymerizable compound (A)-I having a molecular weight of less than 500 and a polyfunctional (meth)acrylic polymerizable compound (A)-II having a molecular weight of 500 or more, and the photopolymerization initiator (B) has a molecular weight of 400 or more and comprises two or more carbonyl groups within a single molecule.

Still another certain preferred embodiment is, for example, a stereolithographic resin composition in which the polyfunctional (meth)acrylic polymerizable compound (A) comprises a polyfunctional (meth)acrylic polymerizable compound (A)-I having a molecular weight of less than 500 and a polyfunctional (meth)acrylic polymerizable compound (A)-II having a molecular weight of 500 or more, and in which the photopolymerization initiator (B) comprises two or more carbonyl groups within a single molecule, and the heteroatoms of the photopolymerization initiator (B) are oxygen atoms and phosphorus atoms.

Yet another certain preferred embodiment is, for example, a stereolithographic resin composition in which the polyfunctional (meth)acrylic polymerizable compound (A) comprises a polyfunctional (meth)acrylic polymerizable compound (A)-I having a molecular weight of less than 500, and the photopolymerization initiator (B) comprises two or more carbonyl groups within a single molecule,

wherein the content of the polyfunctional (meth)acrylic polymerizable compound (A) is 40 to 90 mass% in total 100 mass% of the stereolithographic resin composition, and

the content of the polyfunctional (meth)acrylic polymerizable compound (A)-I is 35 to 50 mass% in total 100 mass% of the stereolithographic resin composition.

Another preferred embodiment is, for example, a stereolithographic resin composition in which the polyfunctional (meth)acrylic polymerizable compound (A) comprises a polyfunctional (meth)acrylic polymerizable compound (A)-I having a molecular weight of less than 500 and a polyfunctional (meth)acrylic polymerizable compound (A)-II having a molecular weight of 500 or more, and in which the photopolymerization initiator (B) comprises two or more carbonyl groups within a single molecule, and the heteroatoms of the photopolymerization initiator (B) are oxygen atoms and phosphorus atoms,

wherein the content of the polyfunctional (meth)acrylic polymerizable compound (A) is 40 to 90 mass% in total 100 mass% of the stereolithographic resin composition, and
the content of the polyfunctional (meth)acrylic polymerizable compound (A)-I is 35 to 50 mass% in total 100 mass% of the stereolithographic resin composition.

In all of the preferred embodiments described above, the type and content of each component may be varied as appropriate following the descriptions of the present specification.

A stereolithographic resin composition of the present invention may comprise a polymerization accelerator to improve photocurability, provided that it does not hinder the intent and purpose of the present invention.

The polymerization accelerator is not particularly limited. However, considering that a stereolithographic resin composition of the present invention is intended for intraoral applications, preferred for use are polymerization accelerators intended for dental use.

Preferred examples of known polymerization accelerators intended for dental use include amines. Examples of amines include aliphatic amines and aromatic amines. The polymerization accelerator may be used alone, or two or more thereof may be used in combination.

Examples of the aromatic amines include ethyl 4-(N,N-dimethylamino)benzoate, methyl 4-(N,N-dimethylamino) benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. In view of imparting superior curability to the stereolithographic resin composition, preferred for use is at least one selected from the group consisting of ethyl 4-(N,N-dimethylamino)benzoate, n-butoxyethyl 4-(N,N-dimethylamino)benzoate, and 4-(N,N-dimethylamino)benzophenone.

In a stereolithographic resin composition of the present invention, a filler may be additionally incorporated to adjust paste properties or to improve the mechanical strength of the shaped article of the stereolithographic resin composition.

Examples of the filler include organic fillers, inorganic fillers, and organic-inorganic composite fillers. The filler may be used alone, or two or more thereof may be used in combination.

Examples of organic filler materials include polymethyl methacrylate, polyethyl methacrylate, a methyl methacrylate-ethyl methacrylate copolymer, crosslinked polymethyl methacrylate, crosslinked polyethyl methacrylate, polyesters, polyamides, polycarbonates, polyphenylene ethers, polyoxymethylene, polyvinyl chloride, polystyrene, polyethylene, polypropylene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. These may be used alone, or two or more thereof may be used in combination. There is no particular restriction on the shape of organic filler, and the particle diameter of the filler may be appropriately selected for use.

Examples of inorganic filler materials include quartz, silica, alumina, silica-titania, silica-titania-barium oxide, silica-zirconia, silica-alumina, lanthanum glass, borosilicate glass, soda glass, barium glass, strontium glass, glass-ceramic, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, and strontium calcium fluoroaluminosilicate glass. These may be used alone, or two or more thereof may be used in combination.

There is no particular restriction on the shape of inorganic filler, and the inorganic filler may be appropriately selected from inorganic fillers of different shapes, such as irregularly shaped fillers and spherical fillers.

The content of inorganic fillers is not particularly limited, provided that it does not hinder the intent and purpose of the present invention. However, for considerations such as possible embrittlement of dental materials such as denture base materials and dental occlusal splints, or materials for treating sleep disorder, the content of inorganic fillers is typically 50 parts or less by mass, preferably 25 parts or less by mass, more preferably 10 parts or less by mass relative to total 100 parts by mass of the polymerizable compounds.

The content of the fillers (for example, inorganic fillers) is preferably 10 mass% or less, more preferably less than 5 mass%, even more preferably less than 1 mass% in total 100 mass% of the stereolithographic resin composition.

A stereolithographic resin composition of the present invention may comprise a polymer to alter properties such as flexibility and flowability, provided that it does not hinder the intent and purpose of the present invention. Examples include natural rubber, synthetic polyisoprene rubber, liquid polyisoprene rubber and hydrogenated products thereof, polybutadiene rubber, liquid polybutadiene rubber and hydrogenated products thereof, styrene-butadiene rubber, chloroprene rubber, ethylene-propylene rubber, acryl rubber, isoprene-isobutylene rubber, acrylonitrile-butadiene rubber, and styrene elastomers. Specific examples of other polymers that may be added include a polystyrene-polyisoprene-polystyrene block copolymer, a polystyrene-polybutadiene-polystyrene block copolymer, a poly($\alpha$-methylstyrene)-polybutadiene-poly($\alpha$-methylstyrene) block copolymer, a poly(p-methylstyrene)-polybutadiene-poly(p-methylstyrene) block copolymer, and hydrogenated products of these.

A stereolithographic resin composition of the present invention may optionally comprise a softener. Examples of

the softener include petroleum-based softeners such as paraffinic, naphthenic, and aromatic process oils, and vegetable oil-based softeners such as paraffin, peanut oil, and rosin. These softeners may be used alone, or two or more thereof may be used in combination. The softener content is not particularly limited, provided that it does not hinder the intent and purpose of the present invention. Typically, the softener content is 200 parts or less by mass, preferably 100 parts or less by mass relative to total 100 parts by mass of the polymerizable compounds.

A stereolithographic resin composition of the present invention may comprise a known stabilizer, in order to inhibit deterioration or adjust photocurability. Examples of such stabilizers include polymerization inhibitors, ultraviolet absorbers, and antioxidants. The stabilizers may be used alone, or two or more thereof may be used in combination.

Examples of the polymerization inhibitors include hydroquinone, hydroquinone monomethyl ether, dibutylhydroquinone, dibutylhydroquinone monomethyl ether, 4-t-butyl catechol, 2-t-butyl-4,6-dimethylphenol, 2,6-di-t-butylphenol, and 3,5-di-t-butyl-4-hydroxytoluene.

The content of the polymerization inhibitors is preferably 0.001 to 5.0 parts by mass relative to total 100 parts by mass of the polymerizable compounds.

A stereolithographic resin composition of the present invention may comprise a known additive, in order to adjust color tones or paste properties. Examples of such additives include pigments, dyes, organic solvents, and thickeners. The additives may be used alone, or two or more thereof may be used in combination.

A stereolithographic resin composition of the present invention exhibits excellent strength, toughness, and water resistance in the shaped article while reducing leachables, in addition to possessing shapeability. This makes a stereolithographic resin composition of the present invention usable in applications where such advantages can be exploited, including intraoral applications. Examples of intraoral applications include dental materials such as denture base materials and dental occlusal splints; and materials for treating sleep disorder (particularly, appliances for the treatment of sleep apnea). A stereolithographic resin composition of the present invention is particularly suited for denture base materials, dental occlusal splints, and appliances for the treatment of sleep apnea.

A shaped article using a stereolithographic resin composition of the present invention may have a shape that depends on intended use. In a stereolithographic resin composition of the present invention, the type and content of each component (polyfunctional (meth)acrylic polymerizable compound (A), photopolymerization initiator (B), and, optionally, monofunctional (meth)acrylic polymerizable compound (C), as well as other optional components such as polymerization accelerators, fillers, polymers, softeners, stabilizers, and additives) may be optionally adjusted for different uses, for example, denture base materials, dental occlusal splints, and appliances for the treatment of sleep apnea.

A stereolithographic resin composition of the present invention can be used in a wide variety of applications by taking advantage of its properties, specifically, the superior dimensional accuracy due to the low rate of volume shrinkage upon curing with light, and the ability to produce shaped products, three-dimensional shaped articles, or other shaped articles that exhibit superior strength, toughness, and water resistance in cured form. For example, a stereolithographic resin composition of the present invention can be used for stereolithographical production of three-dimensional shaped articles, and production of various shaped products, for example, film-shaped articles or molded articles produced by a technique such as film casting or casting, and molds for coating and vacuum molding applications.

A stereolithographic resin composition of the present invention is particularly suited for stereolithography such as above. In stereolithography applications, a stereolithographic resin composition of the present invention enables smooth production of a three-dimensional shaped article having superior toughness and mechanical characteristics while ensuring superior dimensional accuracy with a maintained low rate of volume shrinkage at the time of curing with light.

Another embodiment of the present invention is a method for producing a three-dimensional shaped article by a stereolithography method using any of the stereolithographic resin compositions described above.

The stereolithography method is not particularly limited, and may employ vat photopolymerization techniques such as laser SLA (Stereolithography Apparatus), and DLP (digital light processing) SLA. An example of applicable SLA techniques is LFS (Low Force Stereolithography).

In stereolithography using a stereolithographic resin composition of the present invention, any known stereolithography method and device (for example, a stereolithography device such as the DIGITALWAX® 028J-Plus manufactured by DWS) may be used. In the present invention, the light energy used to cure the resin is preferably an active energy beam. As used herein, "active energy beam" means an energy ray capable of curing a stereolithographic resin composition, and includes, for example, ultraviolet light, an electron beam, X-rays, radiant rays, and high-frequency waves. For example, the active energy beam may be ultraviolet light of 300 to 400 nm wavelengths. The light source of active energy beam may be, for example, a laser such as an Ar laser or a He-Cd laser; or a lighting such as a halogen lamp, a xenon lamp, a metal halide lamp, an LED, a mercury lamp, or a fluorescent lamp. Lasers are particularly preferred. When the light source is a laser, the fabrication time can be reduced by increasing the energy level, and a three-dimensional shaped article of high shape accuracy can be obtained by taking advantage of the desirable convergence of a laser beam.

Stereolithography using a stereolithographic resin composition of the present invention may employ any known method and any known stereolithography system, and the method and device are not particularly limited, as noted above. However, a typical example of a stereolithography method preferred for use in the present invention is a method that

produces the desired final three-dimensional shaped article through a repeated procedure that includes a step of forming a cured layer by selectively applying an active energy beam to the stereolithographic resin composition so as to obtain a cured layer having a desired pattern, and a step of continuously forming another cured layer on the previously formed cured layer by similarly applying an active energy beam to a newly supplied, uncured liquid of the stereolithographic resin composition. The resulting three-dimensional shaped article may be used as is, or, depending on the application, it may be used after improving mechanical characteristics, shape stability, or other properties by, for example, post-curing the product under applied light or heat.

The three-dimensional shaped article obtained by stereolithography is not limited to a particular structure, shape, or size, and these may be decided according to use. Typical examples of areas to which the stereolithography method of the present invention is applicable include production of various models and molds, including, for example, models for assessing external designs in a designing process; models for checking functions of components or parts; resin molds for making casting molds; base models for making molds; and direct molds for prototype molds. More specifically, the stereolithography method of the present invention is applicable to, for example, production of models or work models for precision components and parts, electrical/electronic components, furniture, architectural structures, automobile parts, various containers and vessels, castings, molds, and masters. By taking advantage of the excellent strength and toughness of the shaped article of the stereolithographic resin composition, the stereolithography method of the present invention can be very effectively used for, for example, complex-shape cushioning materials in structures (for example, architectural structures), and molds for vacuum molding.

In view of comfort and usability during use as a denture base material or a dental occlusal splint, a stereolithographic resin composition of the present invention has a flexural modulus ranging preferably from 200 to 3,000 MPa, more preferably from 400 to 2,500 MPa, even more preferably from 600 to 2,000 MPa in cured form.

A stereolithographic resin composition of the present invention has a flexural strength of preferably 15 to 120 MPa, more preferably 20 to 100 MPa, even more preferably 25 to 90 MPa in cured form.

The methods of measurement of flexural modulus and flexural strength are as described in the EXAMPLES below.

EXAMPLES

The following describes the present invention in greater detail by way of Examples. It should be noted, however, that the present invention is in no way limited by the following Examples, and various changes may be made by a person with ordinary skill in the art within the technical idea of the present invention.

The components used for the stereolithographic resin compositions according to Examples and Comparative Examples are described below, along with the abbreviations.

[Polyfunctional (Meth)Acrylic Polymerizable Compound (A)-I-1]

UDMA: 2,2,4-trimethylhexamethylenebis(2-carbamoyloxyethyl)dimethacrylate (manufactured by Kyoeisha Chemical Co., Ltd.; molecular weight: 471) [Polyfunctional (Meth)Acrylic Polymerizable Compound (A)-I-2 Containing no Urethane Bond]

D2.6E: 2,2-bis(4-methacryloyloxypolyethoxyphenyl)propane (with an average of 2.6 moles of ethoxy group added; BPE-100 manufactured by Shin-Nakamura Chemical Co., Ltd.; molecular weight: approximately 478)

[Polyfunctional (Meth)Acrylic Polymerizable Compound (A)-II-1]

<Synthesis Example 1> [Production of Polyfunctional Urethanized (Meth)Acrylic Polymerizable Compound UA1]

(1) A 5 L four-neck flask equipped with a stirrer, a thermostat, a thermometer, and a condenser was charged with 250 g of isophorone diisocyanate and 0.15 g of di-n-butyltin dilaurate, and the mixture was heated to 70°C while being stirred.

(2) Separately, 2,500 g of polyester polyol (Kuraray Polyol® P-2050 manufactured by Kuraray Co., Ltd.; a polyol composed of sebacic acid and 3-methyl-1,5-pentanediol; weight-average molecular weight Mw: 2,000) was added into a dropping funnel equipped with a side tube, and the solution in the dropping funnel was dropped into the flask of (1). Here, the solution was dropped at a constant rate over a time period of 4 hours with the temperature inside the flask held at 65 to 75°C while stirring the solution in the flask of (1). After dropping, the mixture was stirred at the same

temperature for 2 hours to allow reaction.
(3) Thereafter, a homogenous solution prepared by adding 150 g of 2-hydroxyethyl acrylate and 0.4 g of hydroquinone monomethyl ether into a different dropping funnel was dropped into the flask of (2) at a constant rate over a time period of 2 hours with the temperature inside the flask held at 55 to 65°C, and a reaction was allowed for 4 hours at the maintained solution temperature of 70 to 80°C in the flask to obtain a urethanized (meth)acrylic polymerizable compound UA1. The polyfunctional urethanized (meth)acrylic polymerizable compound UA1 had a weight-average molecular weight Mw of 2,600 as measured by GPC analysis.

The weight-average molecular weight Mw of the compound synthesized above means a weight-average molecular weight in terms of polystyrene equivalents, as determined by gel permeation chromatography (GPC).
UA2: N,N'-(2,2,4-trimethylhexamethylene)bis[2-(aminocarboxy)propane-1,3-diol]tetramethacrylate (manufactured by Kyoeisha Chemical Co., Ltd.; molecular weight: 673)

[Polyfunctional (Meth)Acrylic Polymerizable Compound Containing no Urethane Bond (A)-II-2]

Bis-GMA: 2,2-bis[4-(2-hydroxy-3-methacryloyloxypropoxy)phenyl]propane (manufactured by Shin-Nakamura Chemical Co., Ltd.; molecular weight: 513)

[Photopolymerization Initiator (B)]

BAPO: bis(2,4,6-trimethylbenzoyl)phenylphosphine oxide (Omnirad 819 manufactured by IGM Resins B.V. (NL); number of heteroatoms: 4; molecular weight: 418)
OX1: 1,2-octanedione 1-[4-(phenylthio)phenyl]-2-(o-benzoyloxime) (Irgacure OXE-01 manufactured by BASF Japan; number of heteroatoms: 5; molecular weight: 445)
TEO: ethyl phenyl(2,4,6-trimethylbenzoyl)phosphinate (manufactured by Tokyo Chemical Industry Co., Ltd.; number of heteroatoms: 4; molecular weight: 316)

[Photopolymerization Initiators Other Than Photopolymerization Initiator (B)]

TPO: diphenyl(2,4,6-trimethylbenzoyl)phosphine oxide (number of heteroatoms: 3; molecular weight: 348)
TMO: (2,4,6-trimethylbenzoyl)bis(p-tolyl)phosphine oxide (Sinocure 2425 manufactured by SINOCURE Chemical Group; number of heteroatoms: 3; molecular weight: 376)

[Monofunctional (Meth)Acrylic Polymerizable Compound (C)]

POBA: m-phenoxybenzyl acrylate (manufactured by Kyoeisha Chemical Co., Ltd.)
CTMPFA: cyclic trimethylolpropane formal acrylate (manufactured by SARTOMER)
IBA: isobornyl acrylate (manufactured by Kyoeisha Chemical Co., Ltd.)

[Polymerization Inhibitor]

BHT: 3,5-di-t-butyl-4-hydroxytoluene

[Examples 1 to 13 and Comparative Examples 1 to 7]

The components were mixed at an ordinary temperature (20°C ± 15°C; JIS (Japanese Industrial Standards) Z 8703:1983) in the amounts (parts by mass) shown in Tables 1 and 2 to prepare pastes representing the stereolithographic resin compositions according to Examples 1 to 13 and Comparative Examples 1 to 7.

<Shape Accuracy>

The composition indicated in Tables 1 and 2 for each Example and Comparative Example was fabricated into a

single cube-shaped article measuring 10.0 mm on each side, using a stereolithography device (DIGITALWAX® 028J-Plus manufactured by DWS) at a pitch of 50 μm with the light exposure time set to 0.6 seconds per layer. After fabrication, the shaped article was washed with methanol to remove unpolymerized monomers, and the dimensions (unit: mm) were measured in the x-, y-, and z-axis directions using a micrometer. The arithmetic mean of the three measured values along these directions was then calculated, and the shaping error was determined using the equation below. A shaping error of 5.0% or less, as determined by this method, indicates excellent shape accuracy, facilitating good fit in shaped articles such as aligners and denture bases. The results are presented in Tables 1 and 2.

$$\text{Shaping error (\%)} = |(\text{measured dimension}) - 10.0| \times 100$$

<Strength (Flexural Modulus, Flexural Strength) and Toughness (Displacement of Fracture Point)>

The stereolithographic resin composition produced was fabricated into a test specimen with the dimensions (64.0 mm in length, 10.0 mm in width, and 3.3 mm in thickness) described in JIS T 6501:2012 (Acrylic Denture Base Resins), using a stereolithography device (DIGITALWAX® 020D manufactured by DWS) at a pitch of 100 μm with the light exposure time set to 1.3 seconds per layer. A flexure test was conducted using this specimen as a shaped article of the stereolithographic resin composition.

The shaped articles of the stereolithographic resin compositions according to Examples and Comparative Examples were stored in air for 1 day, and measured for strength (flexural modulus, flexural strength) and toughness (displacement of fracture point). The measurements were conducted using a universal testing machine (Autograph AG-I 100kN, manufactured by Shimadzu Corporation) at a crosshead speed of 5 mm/min (n = 5). The arithmetic mean values of the measurements are presented in Tables 1 and 2.

In view of comfort and usability during use as a denture base material or a dental occlusal splint, the preferred flexural modulus range is 200 to 3,000 MPa, more preferably 400 to 2,500 MPa, even more preferably 600 to 2,000 MPa.

The preferred flexural strength is 15 to 120 MPa, more preferably 20 to 100 MPa, even more preferably 25 to 90 MPa.

As for the displacement of fracture point, it is preferable to have no fracture. Specimens were rated "S" (excellent toughness) when there was no fracture, "A" (favorable toughness) when fracture occurred at a displacement of 15 mm or more, "B" (moderate toughness) when fracture occurred at a displacement of 10 mm or more, and "C" (poor toughness) when fracture occurred at a displacement of less than 10 mm, with ratings "S", "A", and "B" considered acceptable.

<Water Resistance>

The stereolithographic resin composition produced was fabricated into a test specimen (64.0 mm in length, 10.0 mm in width, 3.3 mm in thickness), following the same method employed in the flexure test. For the measurement of water resistance, a flexure test was conducted using this specimen as a shaped article of the stereolithographic resin composition.

The shaped articles of the stereolithographic resin compositions according to Examples and Comparative Examples were immersed in 37°C water for 168 hours, and measured for flexural strength in the same manner as in the flexural strength test above (n = 5). The arithmetic mean values of the measurements are presented in Tables 1 and 2. Assuming that the measured flexural strength in the toughness evaluation above is the initial flexural strength, a rate of change (percentage decrease) in flexural strength of 10% or less indicates excellent water resistance, as indicated below. In Tables 1 and 2, the flexural strength after immersion in 37°C water for 168 hours is denoted as "Flexural strength after immersion."

Rate of change (percentage decrease) in flexural strength (%) = [{initial flexural strength (MPa) - flexural strength after immersion in 37°C water for 168 hours (MPa)} / initial flexural strength (MPa)] × 100

<Leachability>

The stereolithographic resin composition produced was fabricated into a disc (15 mm in diameter, 1.0 mm in thickness), using a stereolithography device (DIGITALWAX® 020D manufactured by DWS) at a pitch of 50 μm with the light exposure time set to 0.5 seconds on average per layer. A single disc was then immersed in 2.0 ml of ethanol in a 10 ml glass vial at 50°C for 72 hours. After immersion, the disc was removed, and the ethanol was distilled away before measuring the mass of the resulting extract (n = 1). An amount of leachables less than or equal to 1.0 mg was evaluated as indicating a low level of leachables. The results are presented in Tables 1 and 2.

[Table 1]

| Components (parts by mass) | | | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 |
|---|---|---|---|---|---|---|---|---|---|---|---|---|---|---|---|---|
| | (A)-I | UDMA | | 45 | | 45 | 45 | 45 | 45 | 45 | 45 | 60 | 40 | 40 | 20 | 60 |
| | | D2.6E | | | 45 | | | | | | | | | 5 | 25 | |
| | (A)-II | UA1 | | 35 | 35 | | | 35 | 35 | 35 | 35 | 30 | 25 | 30 | 15 | |
| | | UA2 | | | | 35 | | | | | | | | | | |
| | | Bis-GMA | | | | | 35 | | | | | | | 5 | 20 | |
| | (C) | POBA | | 20 | 20 | 20 | 20 | | | 20 | 20 | 10 | 35 | 20 | 20 | 40 |
| | | CTMPFA | | | | | | 20 | | | | | | | | |
| | | IBA | | | | | | | 20 | | | | | | | |
| | (B) | BAPO | | 1.5 | 1.5 | 1.5 | 0.9 | 1.5 | 1.5 | | | 1.5 | 1.5 | 1.5 | 1.5 | 1.5 |
| | | OX1 | | | | | | | | 1.5 | | | | | | |
| | | TEO | | | | | | | | | 2.0 | | | | | |
| | | BHT | | 0.5 | 0.5 | 0.5 | 0.1 | 0.5 | 0.5 | 0.5 | 0.5 | 0.5 | 0.5 | 0.5 | 0.5 | 0.5 |
| Properties | Shape precision | | (%) | 1.2 | 2.8 | 3.2 | 4.1 | 1.6 | 1.8 | 3.8 | 2.4 | 1.5 | 1.3 | 1.9 | 2.2 | 1.3 |
| | Strength | Flexural strength | (MPa) | 27 | 28 | 88 | 86 | 24 | 21 | 26 | 23 | 32 | 26 | 27 | 48 | 86 |
| | | Flexural modulus | (MPa) | 680 | 710 | 1500 | 1900 | 580 | 390 | 650 | 580 | 830 | 660 | 770 | 1200 | 1800 |
| | Toughness | Displacement of fracture point | | S | A | B | B | S | S | S | S | A | S | S | A | B |
| | Water resistance | Flexural strength after immersion | (MPa) | 26 | 27 | 86 | 79 | 22 | 19 | 25 | 21 | 30 | 25 | 26 | 44 | 81 |
| | | Percentage decrease | (%) | 3.7 | 3.6 | 2.2 | 8.1 | 8.3 | 9.5 | 3.8 | 8.7 | 6.3 | 3.8 | 3.7 | 8.3 | 5.8 |
| | Leachability | Amount of leachables | mg | 0.2 | 0.2 | 0.2 | 0.3 | 0.4 | 0.5 | 0.5 | 0.8 | 0.1 | 0.5 | 0.3 | 0.6 | 0.2 |

[Table 2]

| Components (parts by mass) | | | | Com. Ex. 1 | Com. Ex. 2 | Com. Ex. 3 | Com. Ex. 4 | Com. Ex. 5 | Com. Ex. 6 | Com. Ex. 7 |
|---|---|---|---|---|---|---|---|---|---|---|
| | (A)-I | UDMA | | 45 | | 45 | 60 | 45 | 45 | 45 |
| | | D2.6E | | | 45 | | | | | |
| | (A)-II | UA1 | | 35 | 35 | | | 35 | 35 | 35 |
| | | UA2 | | | | | | | | |
| | | Bis-GMA | | | | 35 | | | | |
| | (C) | POBA | | 20 | 20 | 20 | 40 | 20 | 20 | 20 |
| | | CTMPFA | | | | | | | | |
| | | IBA | | | | | | | | |
| | (B) | BAPO | | | | | | | 0.5 | 4.0 |
| | | OX1 | | | | | | | | |
| | | TPO | | 1.5 | 1.5 | 0.8 | 1.5 | | | |
| | | TMO | | | | | | 1.5 | | |
| | | BHT | | 0.5 | 0.5 | 0.1 | 0.5 | 0.5 | 0.5 | 0.5 |
| Properties | Shape precision | | (%) | 1.1 | 2.6 | 3.2 | 1.3 | 1.1 | 12.1 | 8.6 |
| | Strength | Flexural strength | (MPa) | 27 | 28 | 87 | 88 | 28 | 23 | 22 |
| | | Flexural modulus | (MPa) | 690 | 730 | 2100 | 2300 | 720 | 520 | 480 |
| | Toughness | Displacement of fracture point | | S | A | B | B | S | S | S |
| | Water resistance | Flexural strength after immersion | (MPa) | 26 | 27 | 80 | 82 | 27 | 19 | 16 |
| | | Percentage decrease | (%) | 3.7 | 3.6 | 8.0 | 6.8 | 3.5 | 17.0 | 27.0 |
| | Leachability | Amount of leachables | mg | 3.2 | 4.2 | 4.1 | 2.8 | 2.8 | 0.4 | 0.6 |

As indicated in Tables 1 and 2, the stereolithographic resin compositions of Examples 1 to 13 exhibited excellent shape accuracy, and the shaped articles had superior strength, toughness, and water resistance with reduced amounts of leachables. In particular, the stereolithographic resin compositions according to Examples 1 to 13 were superior to the resin compositions of Comparative Examples 6 and 7 in terms of shape accuracy.

The shaped articles of the stereolithographic resin compositions according to Examples 1 to 13 exhibited superior water resistance compared to the resin compositions of Comparative Examples 6 and 7.

The shaped articles of the stereolithographic resin compositions according to Examples 1 to 13 exhibited superior leachability compared to the shaped articles of the resin compositions of Comparative Examples 1 to 5.

By contrasting Example 1 with Comparative Example 1, Example 2 with Comparative Example 2, Example 4 with Comparative Example 3, and Example 13 with Comparative Example 4, it was confirmed that the amount of leachables from the cured product increases with a combination of components lacking the photopolymerization initiator (B) in the stereolithographic resin composition.

By contrasting Example 1 with Comparative Examples 6 and 7, it was confirmed that sufficient shape accuracy and water resistance cannot be achieved with a combination in which the photopolymerization initiator (B) falls outside the range of 0.75 to 3.0 parts by mass relative to total 100 parts by mass of the polymerizable compounds.

INDUSTRIAL APPLICABILITY

A stereolithographic resin composition of the present invention possesses excellent shape accuracy and exhibits superior strength, toughness, and water resistance in the shaped article while reducing leachables from the shaped article. This makes a stereolithographic resin composition of the present invention suited for intraoral applications, such as a variety of dental materials (particularly, denture base materials and dental occlusal splints) or various materials for treating sleep disorder (particularly, appliances for the treatment of sleep apnea).

## Claims

1. A stereolithographic resin composition comprising a polyfunctional (meth)acrylic polymerizable compound (A), and a photopolymerization initiator containing four or more heteroatoms within a single molecule (B),
wherein the content of the photopolymerization initiator containing four or more heteroatoms within a single molecule (B) is 0.75 to 3.0 parts by mass relative to total 100 parts by mass of polymerizable compounds.

2. The stereolithographic resin composition according to claim 1, which further comprises a monofunctional (meth) acrylic polymerizable compound (C).

3. The stereolithographic resin composition according to claim 1 or 2, wherein the polyfunctional (meth)acrylic polymerizable compound (A) comprises a polyfunctional (meth)acrylic polymerizable compound (A)-I having a molecular weight of less than 500.

4. The stereolithographic resin composition according to claim 1 or 2, wherein the polyfunctional (meth)acrylic polymerizable compound (A) comprises a polyfunctional (meth)acrylic polymerizable compound (A)-II having a molecular weight of 500 or more.

5. The stereolithographic resin composition according to claim 1 or 2, wherein the polyfunctional (meth)acrylic polymerizable compound (A) comprises a polyfunctional (meth)acrylic polymerizable compound (A)-I having a molecular weight of less than 500, and a polyfunctional (meth)acrylic polymerizable compound (A)-II having a molecular weight of 500 or more.

6. The stereolithographic resin composition according to claim 1 or 2, wherein the polyfunctional (meth)acrylic polymerizable compound (A) comprises a polyfunctional (meth)acrylic polymerizable compound (A)-I-1 having a molecular weight of less than 500 and containing a urethane bond, and/or a (meth)acrylic polymerizable compound (A)-II-1 having a molecular weight of 500 or more and containing a urethane bond.

7. The stereolithographic resin composition according to claim 1 or 2, wherein the photopolymerization initiator (B) has a molecular weight of 400 or more.

8. The stereolithographic resin composition according to claim 1 or 2, wherein the photopolymerization initiator (B) comprises two or more carbonyl groups within a single molecule.

9. The stereolithographic resin composition according to claim 1 or 2, wherein the photopolymerization initiator (B) comprises bis(2,4,6-trimethylbenzoyl)phenylphosphine oxide.

10. A dental material comprising a shaped article of the stereolithographic resin composition of claim 1 or 2.

11. A denture base material comprising a shaped article of the stereolithographic resin composition of claim 1 or 2.

12. A dental occlusal splint comprising a shaped article of the stereolithographic resin composition of claim 1 or 2.

13. A material for treating sleep disorder comprising a shaped article of the stereolithographic resin composition of claim 1 or 2.

14. A method for stereolithographically producing a three-dimensional shaped article using the stereolithographic resin composition of claim 1 or 2.

**INTERNATIONAL SEARCH REPORT**

International application No.
**PCT/JP2024/039441**

**A. CLASSIFICATION OF SUBJECT MATTER**

***A61K 6/62***(2020.01)i; ***A61C 13/15***(2006.01)i; ***A61C 13/087***(2006.01)i; ***A61K 6/60***(2020.01)i; ***B29C 64/129***(2017.01)i; ***B29C 64/314***(2017.01)i; ***B33Y 70/00***(2020.01)i; ***C08F 290/06***(2006.01)i; ***C08F 299/06***(2006.01)i
FI: A61K6/62; A61C13/087; A61C13/15; A61K6/60; B29C64/129; B29C64/314; B33Y70/00; C08F290/06; C08F299/06

According to International Patent Classification (IPC) or to both national classification and IPC

**B. FIELDS SEARCHED**

Minimum documentation searched (classification system followed by classification symbols)

A61K6/62; A61C13/15; A61C13/087; A61K6/60; B29C64/129; B29C64/314; B33Y70/00; C08F290/06; C08F299/06

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2023/127934 A1 (KURARAY NORITAKE DENTAL INC.) 06 July 2023 (2023-07-06) claims, paragraphs [0003]-[0010], [0064]-[0066], examples 4, 6 | 1-14 |
| X | JP 2021-126854 A (AIR WATER INC.) 02 September 2021 (2021-09-02) claims, paragraph [0029], examples 1-3, 5-10 | 1-14 |
| X | WO 2023/166781 A1 (DIC CORPORATION) 07 September 2023 (2023-09-07) claims, paragraphs [0066]-[0080], examples 1-4, 10-14 | 1-14 |
| X | WO 2023/188464 A1 (DIC CORPORATION) 05 October 2023 (2023-10-05) claims, paragraphs [0082]-[0092], examples | 1-14 |
| X | WO 2021/149520 A1 (MITSUI CHEMICALS, INC.) 29 July 2021 (2021-07-29) claims, paragraphs [0013]-[0022], examples 20-23, 27-30 | 1-14 |
| X | WO 2023/210328 A1 (TOKUYAMA DENTAL CORP.) 02 November 2023 (2023-11-02) claims, paragraphs [0049]-[0057], examples 1-13, 15-21 | 1-14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 January 2025** | **21 January 2025** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

INTERNATIONAL SEARCH REPORT

International application No.
PCT/JP2024/039441

C. DOCUMENTS CONSIDERED TO BE RELEVANT

| Category* | Citation of document, with indication, where appropriate, of the relevant passages | Relevant to claim No. |
|---|---|---|
| X | JP 2019-151699 A (RICOH COMPANY, LTD.) 12 September 2019 (2019-09-12) claims, paragraphs [0024]-[0026], examples 4-5, 8-9 | 1-14 |
| X | WO 2021/173785 A1 (CARBON, INC.) 02 September 2021 (2021-09-02) claims, p. 8, line 13 to p. 9, line 4, examples | 1-14 |
| X | DE 102018130005 A1 (ENDRESS + HAUSER FLOWTEC AG) 28 May 2020 (2020-05-28) patentanspuruche, paragraphs [0050]-[0066], beispiele 2a-7a, 10a | 1-14 |
| X | WO 2017/18525 A1 (KANEKA CORP.) 02 February 2017 (2017-02-02) claims, paragraphs [0040], [0093]-[0096], mixture examples 3, 6-8 | 1-14 |

Form PCT/ISA/210 (second sheet) (July 2022)

**INTERNATIONAL SEARCH REPORT**
**Information on patent family members**

International application No.
**PCT/JP2024/039441**

| Patent document cited in search report | Publication date (day/month/year) | Patent family member(s) | Publication date (day/month/year) |
|---|---|---|---|
| WO 2023/127934 A1 | 06 July 2023 | (Family: none) | |
| JP 2021-126854 A | 02 September 2021 | (Family: none) | |
| WO 2023/166781 A1 | 07 September 2023 | TW 202336048 A | |
| WO 2023/188464 A1 | 05 October 2023 | TW 202337677 A | |
| WO 2021/149520 A1 | 29 July 2021 | US 2023/0091499 A1<br>claims, paragraphs [0036]-[0070], examples 20-23, 27-30<br>EP 4079768 A4 | |
| WO 2023/210328 A1 | 02 November 2023 | (Family: none) | |
| JP 2019-151699 A | 12 September 2019 | US 2019/0270901 A1<br>claims, paragraphs [0063]-[0088], examples 4-5, 8-9 | |
| WO 2021/173785 A1 | 02 September 2021 | US 2023/0095658 A1<br>EP 4110843 A1 | |
| DE 102018130005 A1 | 28 May 2020 | (Family: none) | |
| WO 2017/18525 A1 | 02 February 2017 | US 2018/0147776 A1<br>claims, paragraphs [0049], [0106]-[0109], formulation examples 3, 6-8 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020158417 A
- WO 2021162007 A
- JP 2000159621 A